# FASCICULE DE BREVET EUROPEEN

(11) **EP 2 843 403 B1**
(45) Date de publication et mention de la délivrance du brevet: **16.03.2016**
(21) Numéro de dépôt: 14168900.0
(22) Date de dépôt: 19.05.2014
(51) Int. Cl.: G01N 30/74, A61M 11/00

(54) **Détecteur évaporatif à diffusion de lumière (DEDL) destiné à être couplé à une colonne de chromatographie en phase liquide ou supercritique ainsi que procédé de gestion dynamique automatique d'un tel DEDL**
Verdampfungs-Lichtstreudetektor (ELSD) zur Koppelung an eine Chromatografiesäule in flüssiger oder superkritischer Phase, und automatisches dynamisches Steuerungsverfahren eines solchen ELSD
Evaporative light scattering detector (ELSD) intended for being coupled to a liquid or supercritical chromatography column and method for automatic dynamic management of such an ELSD

(30) Priorité: 03.07.2013 FR 1356476
(43) Date de publication de la demande: 04.03.2015
(73) Titulaire: Sedere SAS, 94141 Alfortville Cedex (FR)
(72) Inventeur: Pennanec, Rodolphe, 94141 Alfortville Cedex (FR); Gangloff, Henry, 94141 Alfortville Cedex (FR)
(74) Mandataire: Cabinet HERRBURGER

(56) Documents cités:
- WO-A1-99/14556
- WO-A2-2006/083511
- GB-A- 2 068 539
- US-A1- 2003 086 092
- US-A1- 2009 122 315

## Description

La présente invention a pour objet un détecteur évaporatif à diffusion de lumière (DEDL) destiné à être couplé à une colonne de chromatographie en phase liquide ou supercritique.

Un tel appareil a pour avantage d'être universel, c'est-à-dire de permettre l'analyse de tous les composés moins volatils que la phase mobile dans laquelle ils sont transportés, en particulier les cations et les anions minéraux ou organiques contenus dans un échantillon, ce sans préparation de cet échantillon ni traitement chimique particulier, sauf éventuellement la transformation de composés volatils en composés non volatils.

Le principe de fonctionnement d'un DEDL est le suivant : les composés à analyser sont transportés par une phase mobile ou un liquide porteur plus volatil qui est ensuite nébulisé puis évaporé à température relativement faible de sorte que seules demeurent des microparticules résiduelles - idéalement les composés à analyser - pouvant être détectées par diffusion de lumière.

On peut ainsi analyser directement des effluents provenant de colonnes de chromatographie en phase liquide ou supercritique à la seule condition de choisir un éluant suffisamment volatil pour être directement utilisé en tant que phase mobile au niveau du DEDL.

De tels DEDL comportent classiquement :
- un nébuliseur en particulier associé à une chambre de nébulisation dans lequel on introduit d'une part un échantillon provenant en règle générale d'une colonne de chromatographie et constitué par un liquide porteur renfermant des composés à analyser non volatils qui ont été dissous dans celui-ci, et d'autre part un gaz de nébulisation permettant de transformer l'échantillon en un aérosol,
- une chambre d'évaporation dans laquelle on évapore le liquide porteur afin de ne conserver que des microparticules des composés à analyser, et
- une chambre de détection dans laquelle sont introduites les microparticules des composés à analyser renfermées dans l'échantillon provenant de la colonne de chromatographie.

Une telle chambre de détection est équipée d'une source permettant l'irradiation des microparticules des composés à analyser introduites dans celle-ci par un rayonnement issu d'une source poly-chromatique ou monochromatique ainsi que d'un dispositif de mesure à commande électronique permettant de détecter la lumière diffusée par ces microparticules dans une direction différente de celle du faisceau d'irradiation et d'obtenir en sortie des signaux électriques notamment numériques proportionnels à l'intensité du flux lumineux reçu par ce dispositif de mesure.

Ces signaux de mesure sont transmis à l'ordinateur d'un utilisateur de façon à permettre d'obtenir sur l'écran de cet ordinateur un chromatogramme V=f(t) comportant une série de pics représentatifs de la présence des composés à analyser et de leur teneur dans l'échantillon provenant de la colonne de chromatographie.

La chambre de détection de la quasi-totalité des DEDL actuellement proposés sur le marché est équipée en tant que dispositif de mesure de la lumière à commande électronique, d'un photomultiplicateur ou d'une photodiode couplé(e) à un convertisseur courant/tension et à un convertisseur analogique/numérique.

Un tel photomultiplicateur ou une telle photodiode travaille en continu mais l'électronique responsable de la conversion analogique/numérique travaille, quant à elle de manière séquentielle et fournit en sortie des signaux à une fréquence de restitution prédéfinie qui peut être contrôlable par l'utilisateur ou peut être imposée par l'électronique afin de fournir à l'utilisateur des signaux à une fréquence choisie par celui-ci.

Après numérisation, ces signaux sont transférés vers l'ordinateur de l'utilisateur de façon à permettre d'obtenir sur l'écran de celui-ci une série de points de mesure (dont le nombre correspond à la fréquence de restitution) qui, après qu'ils aient été reliés conduisent à l'établissement du chromatogramme de l'échantillon à analyser.

Sur ce chromatogramme et dans le cas d'un photomultiplicateur, le gain, et par voie de conséquence la hauteur ou ordonnée intrinsèque de chaque point de mesure est directement proportionnel à la valeur de la haute tension appliquée à ce photomultiplicateur qui peut être réglée par l'utilisateur.

Un tel réglage est en règle générale effectué de manière fixe pour chaque analyse.

Lors de ce réglage, il est nécessaire pour obtenir des chromatogrammes corrects et représentatifs du produit à analyser de tenir compte de deux paramètres qui sont la limite de détection, c'est-à-dire la plus petite quantité de produit à analyser pouvant être mesurée par le détecteur de lumière et la valeur de saturation qui correspond à la limite à partir de laquelle ce détecteur reçoit trop de lumière pour qu'il puisse effectuer une mesure.

Dans les DEDL équipés de photomultiplicateurs actuellement proposés sur le marché, lorsque la valeur de saturation est atteinte au cours d'une analyse, l'utilisateur doit recommencer cette analyse en diminuant la haute tension d'alimentation du photomultiplicateur, ou, en d'autres termes le gain, ce qui entraine une perte de temps et d'échantillon pouvant être très préjudiciables, et parallèlement une diminution de la sensibilité de la mesure du fait que la limite de détection est parallèlement augmentée.

Les DEDL équipés de photomultiplicateurs présentent en outre l'inconvénient d'être relativement onéreux.

Pour remédier à ce dernier inconvénient, il a été envisagé de remplacer le photomultiplicateur équipant la chambre de détection de ces DEDL par un autre élément de mesure de la lumière moins onéreux, à savoir une photodiode.

Une photodiode ne permet toutefois pas à l'utilisateur de régler le gain, et par suite la hauteur ou ordonnée intrinsèque des points de mesure permettant l'établissement du chromatogramme, de sorte que la simple substitution d'un photomultiplicateur par une photodiode en conservant la maitrise du gain n'est pas possible.

Le document WO2006083511 (WATERS INVESTMENTS LTD, publié le 10-08-2006) est considéré comme l'état de la technique le plus proche de la présente invention et divulgue un détecteur évaporatif à diffusion de lumière (DEDL) destiné à être couplé à une colonne de chromatographie en phase liquide ou supercritique et comportant un nébuliseur en particulier associé à une chambre de nébulisation, une chambre d'évaporation et une chambre de détection dans laquelle sont introduites des microparticules de composés à analysés renfermés dans un échantillon provenant de la colonne de chromatographie, cette chambre de détection étant équipée d'une source d'irradiation des microparticules des composés à analyser introduites dans celle-ci ainsi que d'un dispositif de mesure à commande électronique permettant de détecter la lumière diffusée par ces microparticules dans une direction différente de celle du faisceau d'irradiation et d'obtenir en sortie des signaux électriques notamment numériques proportionnels à l'intensité du flux lumineux reçu par ce dispositif de mesure, ces signaux de mesure étant transmis à l'ordinateur d'un utilisateur de façon à permettre d'obtenir sur l'écran de cet ordinateur un chromatogramme comportant une série de pics représentatifs de la nature des composés à analyser et de leur teneur dans l'échantillon provenant de la colonne de chromatographie.

La présente invention a pour objet de proposer un DEDL destiné à être couplé à une colonne de chromatographie en phase liquide ou supercritique du type susmentionné de nature à permettre de remédier à cet inconvénient.

Selon l'invention, un tel DEDL est caractérisé en ce que le dispositif de mesure de la lumière à commande électronique équipant sa chambre de détection comporte un détecteur de lumière, en particulier une photodiode, couplé à un organe de gestion dynamique automatique à intégration notamment équipé d'une série de condensateurs de capacité définie pouvant être commutés individuellement de façon réglable et remplissant la fonction d'un convertisseur analogique/numérique à entrée en courant et sortie numérique permettant d'accumuler le courant délivré en sortie du détecteur de lumière, en particulier de la photodiode, pendant une très courte durée pouvant être réglée automatiquement en interne par un micro-logiciel de pilotage.

Il est à noter que si le détecteur de lumière est en règle générale une photodiode, il n'est pas exclu de remplacer celle-ci par un photomultiplicateur également couplé à un organe d'entraînement de gestion dynamique à intégration sans pour cela sortir du cadre de l'invention.

Il est donc ainsi possible de superposer à la fréquence de restitution, c'est-à-dire à la fréquence de transfert des signaux de mesure vers l'ordinateur de l'utilisateur une fréquence secondaire ou fréquence d'acquisition du signal délivré en sortie de l'organe de gestion dynamique automatique à intégration qui est nettement supérieure et est une fonction directe de la durée d'accumulation du courant obtenu en sortie du détecteur de lumière, en particulier de la photodiode qui peut être assimilée à un gain.

Cette superposition se traduit au niveau du chromatogramme obtenu sur l'écran de l'ordinateur de l'utilisateur par l'obtention de points de mesure dont la hauteur ou ordonnée intrinsèque est d'autant plus grande que la durée d'accumulation (c'est-à-dire le gain) est importante.

Par suite, une modification de la durée d'accumulation de l'organe de gestion dynamique automatique se traduit au niveau du chromatogramme par un comportement pouvant être assimilé à une modification du gain d'un photomultiplicateur.

De tels organes de gestion dynamique automatique à intégration jouant le rôle de convertisseur analogique/numérique à entrée en courant et à accumulation de courant correspondent à titre d'exemple aux DDC 112 ou DDC 114 actuellement proposés dans le commerce.

La présente invention a également pour objet un procédé de gestion dynamique automatique d'un DEDL ayant les caractéristiques susmentionnées.

Selon ce procédé on règle automatiquement la durée d'accumulation de l'organe de gestion dynamique en fonction de la valeur des signaux de courant délivrés en sortie du détecteur de lumière, en particulier de la photodiode de sorte que cette durée soit réglée à une valeur élevée lorsque la valeur du signal est faible, c'est-à-dire proche de la limite de détection de façon à se placer dans des conditions de sensibilité de détection optimales, et soit réglée à une valeur plus faible lorsque la valeur de ce signal augmente et s'approche de la limite de saturation pour la du- - rée d'accumulation utilisée.

De façon plus précise et selon une autre caractéristique de l'invention, on rentre préalablement dans le micro-logiciel de pilotage de l'organe de gestion dynamique automatique à intégration un signal de seuil haut un peu inférieur à la limite de saturation et correspondant en sortie à une tension de seuil haute ainsi qu'un signal de seuil bas correspondant en sortie à une tension de seuil basse, et lorsque dans la partie ascendante d'un pic la valeur de mesure atteint la tension de seuil haute pour la durée d'accumulation utilisée on diminue automatiquement cette durée d'accumulation de façon à s'éloigner de la limite de saturation, et inversement lorsque dans la partie descendante d'un pic la valeur de mesure atteint la tension de seuil basse pour la durée d'accumulation utilisée ou augmente automatiquement cette durée d'accumulation de façon à se placer dans de meilleures conditions de sensibilité de détection.

Un tel procédé permet par suite de garantir dans tous les cas un maximum de sensibilité dans la mesure où dans le cas des signaux les plus faibles, c'est-à-dire proches de la limite de détection, on utilise la durée d'utilisation et donc le gain maximum alors que l'on peut parallèlement limiter le risque de saturer le détecteur en diminuant automatiquement le gain de façon à s'éloigner de la limite de saturation.

En d'autres termes, le procédé conforme à l'invention permet d'augmenter automatiquement, donc sans intervention de l'utilisateur la gamme dynamique apparente du dispositif de mesure de la lumière, c'est-à-dire la plage comprise entre la valeur minimum détectable et la valeur maximum détectable qui correspond à la limite de saturation du détecteur.

Conformément à l'invention, l'augmentation ou la diminu tion automatique de la durée d'accumulation de l'organe de gestion dynamique est avantageusement effectuée par paliers, à chaque fois d'un facteur défini qui est de préférence un facteur entier, en particulier un facteur 2.

Selon une autre caractéristique préférentielle de l'invention, liée au fait que la modification de la durée d'accumulation est effectuée de manière quantitative, c'est-à-dire qu'une relation directe est conservée entre la mesure réellement effectuée et la mesure telle qu'elle aurait été si la valeur de la durée d'accumulation n'avait pas été modifiée, on restitue sur l'écran de l'ordinateur de l'utilisateur un chromatogramme identique à celui qui aurait été théoriquement obtenu sans modification de la durée d'accumulation de l'organe de gestion dynamique et en l'absence de limite de saturation.

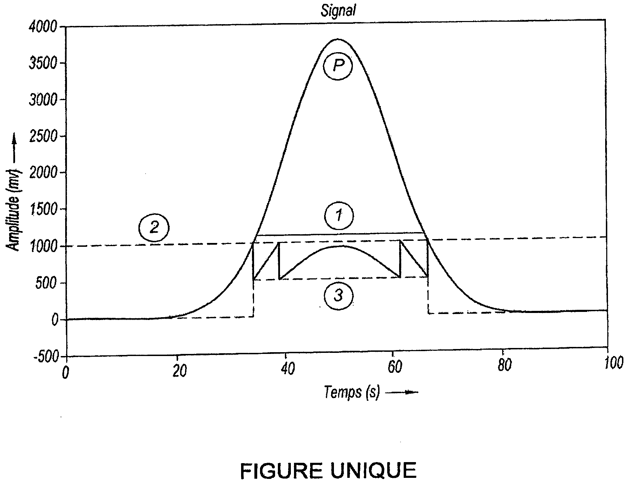

Les caractéristiques du procédé qui fait l'objet de l'invention seront illustrées par la figure annexée à titre d'exemple qui correspond à un chromatogramme théorique constitué par un pic gaussien unique P.

Selon cette figure, la limite de détection du dispositif de mesure de la lumière représentée par la droite horizontale 1 est de 1100 mV.

La tension de seuil haute et la tension de seuil basse préalablement rentrées dans le micro-logiciel de pilotage de l'organe de gestion dynamique automatique à intégration qui sont respectivement égales à 1000 et à 500 mV sont schématisées par les droites horizontales en pointillés 2 et 3.

Conformément à l'invention, dans la partie ascendante du pic P, lorsque le signal de tension atteint la valeur de seuil haute 2 qui est un peu inférieure à la limite de saturation 1, la durée d'accumulation et donc l'ordonnée du signal de mesure est automatiquement diminuée d'un facteur 2.

Le signal de mesure passe donc automatiquement de 1000 à 500 mV et l'enregistrement du pic se poursuit ainsi jusqu'à ce que l'ordonnée du signal de mesure atteigne à nouveau la valeur de seuil haute 2 égale à 1000 mV et soit à nouveau diminuée d'un facteur 2 pour passer à 500 mV.

Il est à noter que selon l'exemple représenté cette valeur de 500 mV correspond incidemment à la valeur de seuil basse.

Dans la partie descendante du pic, le processus inverse est effectué, c'est-à-dire que lorsque le signal de mesure atteint la valeur de seuil basse 3, la durée d'accumulation de l'organe de gestion dynamique est automatiquement augmentée d'un facteur 2.

Le signal de mesure passe donc automatiquement de 500 à 1000 mV et l'enregistrement du pic se poursuit ainsi jusqu'à ce que l'ordonnée du signal de mesure atteigne à nouveau la valeur de seuil basse égale 3 à 500 mV, et soit à nouveau augmentée d'un facteur 2 pour passer à 1000 mV.

Pour reconstituer le pic P au niveau de l'écran de l'ordinateur de l'utilisateur qui correspond à la durée d'utilisation maximum sans tenir compte de la valeur de saturation il suffit de multiplier le signal mesuré par les facteurs correctifs appliqués sur la durée d'accumulation.

## Revendications

1. Détecteur évaporatif à diffusion de lumière (DEDL) destiné à être couplé à une colonne de chromatographie en phase liquide ou supercritique et comportant un nébuliseur en particulier associé à une chambre de nébulisation, une chambre d'évaporation et une chambre de détection dans laquelle sont introduites des microparticules de composés à analysés renfermés dans un échantillon provenant de la colonne de chromatographie, cette chambre de détection étant équipée d'une source d'irradiation des microparticules des composés à analyser introduites dans celle-ci ainsi que d'un dispositif de mesure à commande électronique permettant de détecter la lumière diffusée par ces microparticules dans une direction différente de celle du faisceau d'irradiation et d'obtenir en sortie des signaux électriques notamment numériques proportionnels à l'intensité du flux lumineux reçu par ce dispositif de mesure, ces signaux de mesure étant transmis à l'ordinateur d'un utilisateur de façon à permettre d'obtenir sur l'écran de cet ordinateur un chromatogramme V=f(t) comportant une série de pics représentatifs de la nature des composés à analyser et de leur teneur dans l'échantillon provenant de la colonne de chromatographie, **caractérisé en ce que**
le dispositif de mesure de la lumière à commande électronique équipant la chambre de détection du DEDL comporte un détecteur de lumière, en particulier une photodiode, couplé à un organe de gestion dynamique automatique à intégration notamment équipé d'une série de condensateurs de capacité définie pouvant être commutés individuellement de façon réglable et remplissant la fonction d'un convertisseur analogique/numérique à entrée en courant et sortie numérique permettant d'accumuler le courant délivré en sortie du détecteur de lumière, en particulier de la photodiode pendant une très courte durée pouvant être réglée automatiquement en interne par un micro-logiciel de pilotage.

2. Détecteur évaporatif à diffusion de lumière conforme à la revendication 1,
**caractérisé en ce que**
le détecteur de lumière est une photodiode.

3. Procédé de gestion dynamique automatique d'un DEDL conforme à la revendication 1 ou 2,
**caractérisé en ce que**
l'on règle automatiquement la durée d'accumulation de l'organe de gestion dynamique en fonction de la valeur des signaux de courant délivrés en sortie du détecteur de lumière, en particulier de la photodiode de sorte que cette durée soit réglée à une valeur élevée lorsque la valeur du signal est faible de façon à se placer dans des conditions de sensibilité de détection optima et soit réglée à une valeur plus faible lorsque la valeur de ce signal augmente et s'approche de la limite de saturation pour la durée d'accumulation utilisée.

4. Procédé conforme à la revendication 3,
**caractérisé en ce que**
l'on rentre préalablement dans le micro-logiciel de pilotage de l'organe de gestion dynamique automatique à intégration un signal de seuil haut un peu inférieur à la limite de saturation et correspondant en sortie à une tension de seuil haute ainsi qu'un signal de seuil bas correspondant en sortie à une tension de seuil basse, et lorsque dans la partie ascendante d'un pic la valeur de mesure atteint la tenson de seuil haute pour la durée d'accumulation utilisée on diminue automatiquement cette durée d'accumulation de façon à s'éloigner de la limite de saturation, et inversement, lorsque, dans la partie descendante d'un pic la valeur de mesure atteint la tension de seuil basse pour la durée d'accumulation utilisée, ou augmente automatiquement cette durée d'accumulation de façon à se placer dans de meilleurs conditions de sensibilité de détection.

5. Procédé selon l'une quelconque des revendications 3 et 4,
**caractérisé en ce que**
l'on augmente ou diminue automatiquement la durée d'accumulation de l'organe de gestion dynamique par paliers, à chaque fois d'un facteur défini.

6. Procédé selon la revendication 5,
**caractérisé en ce que**
le facteur défini est un facteur entier, de préférence un facteur 2.

7. Procédé selon l'une quelconque des revendications 3 à 6,
**caractérisé en ce que**
l'on restitue sur l'écran de l'ordinateur de l'utilisateur un chromatogramme identique à celui qui aurait été théoriquement obtenu sans modification de la durée d'accumulation de l'organe de gestion dynamique et en l'absence de limite de saturation.

## Patentansprüche

1. Verdampfungs-Lichtstreudetektor (DEDL), der dazu bestimmt ist, an eine Chromatografiesäule in flüssiger oder superkritischer Phase gekoppelt zu werden und Folgendes umfasst: einen insbesondere mit einer Vernebelungskammer verbunden Vernebler, eine Verdampfungskammer und eine Detektionskammer, in die Mikropartikel von zu analysierenden Verbindungen eingeführt werden, die in einer von der Chromatografiesäule kommenden Probe eingeschlossen sind, wobei diese Detektionskammer mit Folgendem versehen ist: mit einer Quelle zur Bestrahlung der in sie eingeführten Mikropartikel der zu analysierenden Verbindungen und mit einer elektronisch gesteuerten Messvorrichtung, die es ermöglicht, das Licht zu detektieren, das von diesen Mikropartikeln in einer Richtung ausgestrahlt wird, die sich von jener des Bestrahlungsbündels unterscheidet, und am Ausgang insbesondere digitale elektrische Signale zu erhalten, die proportional zur Stärke des von der Messvorrichtung empfangenen Lichtstroms sind, wobei diese Messsignale zu einem Computer eines Benutzers gesendet werden, um am Bildschirm dieses Computers ein Chromatogramm V=f(t) erstellen zu können, das eine Reihe von Spitzen enthält, die repräsentativ für die Art der zu analysierenden Verbindungen und ihren Gehalt in der von der Chromatografiesäule kommenden Probe sind,
**dadurch gekennzeichnet, dass**
die elektronisch gesteuerte Lichtmessvorrichtung, mit der die Detektionskammer des DEDL versehen ist, einen Lichtdetektor, insbesondere eine Fotodiode, umfasst, die mit einem Organ zur integrierten automatischen dynamischen Steuerung gekoppelt ist, das insbesondere mit einer Reihe von Kondensatoren mit definierter Kapazität versehen ist, die einzeln auf einstellbare Weise geschaltet werden können und die Funktion eines Analog-Digital-Wandlers mit Stromeingang und Digitalausgang ausüben, wodurch es möglich ist, den am Ausgang des Lichtdetektors, insbesondere der Fotodiode, abgegebenen Strom während eines sehr kurzen Zeitraums, der intern automatisch durch eine Mikro-Steuersoftware eingestellt werden kann, zu speichern.

2. Verdampfungs-Lichtstreudetektor nach Anspruch 1,
**dadurch gekennzeichnet, dass**
der Lichtdetektor eine Fotodiode ist.

3. Verfahren zur automatischen dynamischen Steuerung eines DEDL nach Anspruch 1 oder 2,
**dadurch gekennzeichnet, dass**
die Speicherdauer des Organs zur dynamischen Steuerung in Abhängigkeit vom Wert der am Ausgang des Lichtdetektors, insbesondere der Fotodiode, ausgegebenen Stromsignale automatisch derart geregelt wird, dass diese Dauer auf einen hohen Wert eingestellt wird, wenn der Wert des Signals niedrig ist, um optimale Bedingungen bezüglich der Detektionsempfindlichkeit herzustellen, und auf einen niedrigeren Wert eingestellt wird, wenn der Wert dieses Signals steigt und sich der Sättigungsgrenze für die verwendete Speicherdauer annähert.

4. Verfahren nach Anspruch 3,
**dadurch gekennzeichnet, dass**
in die Mikro-Steuersoftware des Organs zur integrierten automatischen dynamischen Steuerung vorab Folgendes eingegeben wird: ein oberes Schwellenwertsignal, das geringfügig niedriger als die Sättigungsgrenze ist und am Ausgang einer oberen Schwellenspannung entspricht, und ein unteres Schwellenwertsignal, das am Ausgang einer unteren Schwellenspannung entspricht, wobei, wenn der Messwert im aufsteigenden Teil einer Spitze während der verwendeten Speicherdauer die obere Schwellenspannung erreicht, diese Speicherdauer automatisch verringert wird, um sich von der Sättigungsgrenze zu entfernen, und wobei umgekehrt, wenn der Messwert im absteigenden Teil einer Spitze während der verwendeten Speicherdauer die untere Schwellenspannung erreicht, diese Speicherdauer automatisch erhöht wird, um bessere Bedingungen bezüglich der Detektionsempfindlichkeit herzustellen.

5. Verfahren nach einem der Ansprüche 3 und 4,
**dadurch gekennzeichnet, dass**
die Speicherdauer des Organs zur dynamischen Steuerung jeweils um einen definierten Faktor automatisch stufenweise erhöht oder verringert wird.

6. Verfahren nach Anspruch 5,
**dadurch gekennzeichnet, dass**
der definierte Faktor ein ganzzahliger Faktor, vorzugsweise ein Faktor 2 ist.

7. Verfahren nach einem der Ansprüche 3 bis 6,
**dadurch gekennzeichnet, dass**
auf dem Bildschirm des Computers des Benutzers ein Chromatogramm wiedergegeben wird, das identisch mit jenem ist, das theoretisch ohne Änderung der Speicherdauer des Organs zur dynamischen Steuerung und in Abwesenheit einer Sättigungsgrenze erzielt worden wäre.

## Claims

1. Evaporative light-scattering device (ELSD) for coupling to a liquid chromatography or supercritical fluid chromatography column and comprising a nebuliser in particular associated with a nebulisation chamber, an evaporation chamber and a detection chamber into which there are introduced microparticles of compounds to be analysed which are contained in a sample from the chromatography column, the detection chamber being equipped with a source for irradiation of the microparticles of the compounds to be analysed that are introduced into the detection chamber and also with an electronically controlled measuring device which allows the light scattered by the microparticles in a direction different from that of the irradiation beam to be detected and electrical, especially digital, signals proportional to the intensity of the luminous flux received by the measuring device to be obtained, the measured signals being transmitted to a user's computer so as to allow a chromatogram V=f(t) comprising a series of peaks that are representative of the nature of the compounds to be analysed and of the content thereof in the sample from the chromatography column to be obtained on the computer screen,
**characterised in that**
the electronically controlled light measuring device with which the detection chamber of the ELSD is equipped comprises a light detector, in particular a photodiode, coupled to an integrating automatic dynamic management element especially equipped with a series of capacitors of defined capacity which can be switched individually in an adjustable manner and which perform the function of an analogue/digital converter with current input and digital output allowing the current delivered at the output of the light detector, in particular of the photodiode, to be accumulated for a very short time which can automatically be adjusted internally by driving software.

2. Evaporative light-scattering device according to claim 1,
**characterised in that**
the light detector is a photodiode.

3. Method for automatic dynamic management of an ELSD according to claim 1 or 2,
**characterised in that**
the accumulation time of the dynamic management element is adjusted automatically according to the value of the current signals delivered at the output of the light detector, in particular of the photodiode, so that the time is adjusted to a high value when the value of the signal is low, in order to have optimum detection sensitivity conditions, and is adjusted to a lower value when the value of the signal increases and approaches the saturation limit for the accumulation time used.

4. Method according to claim 3,
**characterised in that**
there are entered beforehand into the software for driving the integrating automatic dynamic management element a high threshold signal slightly below the saturation limit and corresponding at the output to a high threshold voltage, as well as a low threshold signal corresponding at the output to a low threshold voltage, and when, in the ascending portion of a peak, the measured value reaches the high threshold value for the accumulation time used, the accumulation time is automatically reduced so as to move away from the saturation limit, and *vice versa* when, in the descending portion of a peak, the measured value reaches the low threshold voltage for the accumulation time used, or automatically increases the accumulation time in order to have better detection sensitivity conditions.

5. Method according to either claim 3 or claim 4,
**characterised in that**
the accumulation time of the dynamic management element is automatically increased or reduced in steps, by a defined factor each time.

6. Method according to claim 5,
**characterised in that**
the defined factor is a whole factor, preferably a factor of 2.

7. Method according to any one of claims 3 to 6,
**characterised in that**
there is reproduced on the user's computer screen a chromatogram identical to that which would theoretically have been obtained without modification of the accumulation time of the dynamic management element and in the absence of a saturation limit.
